# EUROPEAN PATENT SPECIFICATION

(11) **EP 4 231 308 B1**
(45) Date of publication and mention of the grant of the patent: **15.04.2026**
(21) Application number: 23184674.2
(22) Date of filing: 13.11.2020
(51) Int. Cl.: G16H 20/17, A61B 5/145

(54) **ADAPTIVE DURATION OF INSULIN ACTION FOR USE IN INSULIN ON BOARD CALCULATIONS**
ADAPTIVE DAUER DER INSULINWIRKUNG ZUR VERWENDUNG BEI INSULIN-ON-BOARD-BERECHNUNGEN
DURÉE ADAPTATIVE D'ACTION DE L'INSULINE DESTINÉE À ÊTRE UTILISÉE DANS DES CALCULS D'INSULINE À BORD

(30) Priority: 13.11.2019 US 201962934618 P
(43) Date of publication of application: 23.08.2023
(62) Divisional of application: 20207445.6
(73) Proprietor: Insulet Corporation, Acton, MA 01720 (US)
(72) Inventor: CARDINALI, Steven, Tewksbury (US); LEE, Joon Bok, Acton (US)
(74) Representative: Peterreins Schley

(56) References cited:
- US-A1- 2014 200 426
- US-A1- 2014 276 556
- US-A1- 2016 038 673

## Description

### BACKGROUND

Due to the complicated and dynamic nature of the human body's response to insulin, it is unfortunately common for patients to end up in a hypoglycemic or hyperglycemic state after being treated with insulin therapy. This outcome is undesirable for many reasons: hypoglycemia creates an immediate risk of a severe medical event (seizure, coma, death) while hyperglycemia creates long term negative health effects as well as the risk of ketoacidosis. Whether a person ends up in one of these states depends on a very complicated combination of many factors and sources of error. One source of error comes from the assumed duration of insulin action in the patient. Insulin therapy systems use a value, or function, to determine how long insulin remains active in a patient.

When a patient is treating themselves, a conventional approach of automatic insulin delivery algorithms is to assume a static duration of insulin action that is constant and equal for all patients. In some instances, manual duration of insulin action may be set by a user, which is not optimal. In addition, the static duration of insulin action setting is not adaptive based on user history and requires user intervention and input.

US 2016/038673 A1 discloses a dynamic model for insulin time-action profiles. Various aspects are suitable for implementation in an embedded system such as an insulin pump, or a handheld device such as a pump remote control. Various aspects can be used as an off-line modeling tool running on a smartphone to assist patients in their treatment, or can be used as part of a closed-loop control algorithm for an artificial pancreas. US 2014/276556 A1 discloses a computer implemented method of determining a clinical variable utilizing an insulin pump that includes initiating blood glucose measurements, initiating ingestion of carbohydrates and receiving input data based on the blood glucose measurements and the ingestion of carbohydrates and utilizing the data to calculate clinical variables. The method includes presenting instructions to a patient to take various actions and to input various data. The clinical variables determined may be stored in memory and then used to calculate insulin doses and to send a signal to an insulin pump to infuse the insulin dose calculated.

### SUMMARY

According to an aspect of the invention, a system comprising a management device is disclosed. The device comprises a processor, a memory, a user interface, and a transceiver. The memory stores programming code and is operable to store data, wherein the programming code is executable by the processor. The transceiver is operable to receive and transmit signals containing information usable by or generated by the programming code. The processor when executing the programming code is operable to control delivery of insulin, and to perform functions to: monitor a number of glucose measurements of a user; determine, at predetermined intervals, a value of a user's insulin onboard; in response to a determination that the user's insulin onboard is less than or equal to zero, determine whether a rate of change of the number of glucose measurements is a negative rate of change; as a result of a determination that the rate of change of the number of glucose measurements is negative, modify a confidence value by increasing the confidence value, wherein the confidence value is related to a duration of insulin action setting; compare the modified confidence value to a confidence value threshold; as a result of a determination that the modified confidence value is greater than the confidence value threshold , modify a duration of insulin action setting by increasing the duration of insulin action setting and reset the confidence value; determine timing of an insulin dose based on the modified duration of insulin action setting; and output a command signal to deliver the insulin dose based on the determined timing.

According to another aspect of the invention, a method for operating a management device is disclosed. The method includes: monitoring a number of glucose measurements of a user; determining, at predetermined intervals, a value of a user's insulin onboard; in response to a determination that the user's insulin onboard is less than or equal to zero, determining whether a rate of change of the number of glucose measurements is a negative rate of change; as a result of the determination of the negative rate of change, modifying a confidence value by increasing the confidence value, wherein the confidence value is related to a duration of insulin action setting; comparing the confidence value to a confidence value threshold; as a result of a determination that the modified confidence value is greater than the confidence value threshold, modifying a duration of insulin action setting by increasing the duration of insulin action setting; resetting the confidence value; determining timing of an insulin dose based on the modified duration of insulin action setting; and outputting a command signal to deliver an insulin dose based on the determined timing.

For illustrative purposes there is also disclosed an example of a method. The method includes monitoring a number of blood glucose measurements of a user. A value of a user's insulin onboard may be determined at predetermined intervals. In response to a determination that the user's insulin onboard is less than or equal to zero, whether a rate of change of the number of blood glucose measurements is a negative rate of change may be determined. As a result of the determination, a confidence value, which is related to a duration of insulin action setting, may be modified. A duration of insulin action setting may be modified based on the modified confidence value. The timing of an insulin dose may be determined based on the modified duration of insulin action setting. A command signal to deliver an insulin dose based on the determined timing may be output.

### BRIEF DESCRIPTION OF THE DRAWINGS

FIG. 1 shows a flow chart of an example of a process for updating a duration of insulin action setting.
FIG. 2 shows a flow chart of an example of an alternate process for updating a duration of insulin action setting.
FIG. 3 illustrates a functional block diagram of drug delivery system suitable for implementing the example processes and techniques described herein.
FIG. 4 illustrates an example of a blood glucose measurements (upper chart) and insulin delivery (lower chart).

### DETAILED DESCRIPTION

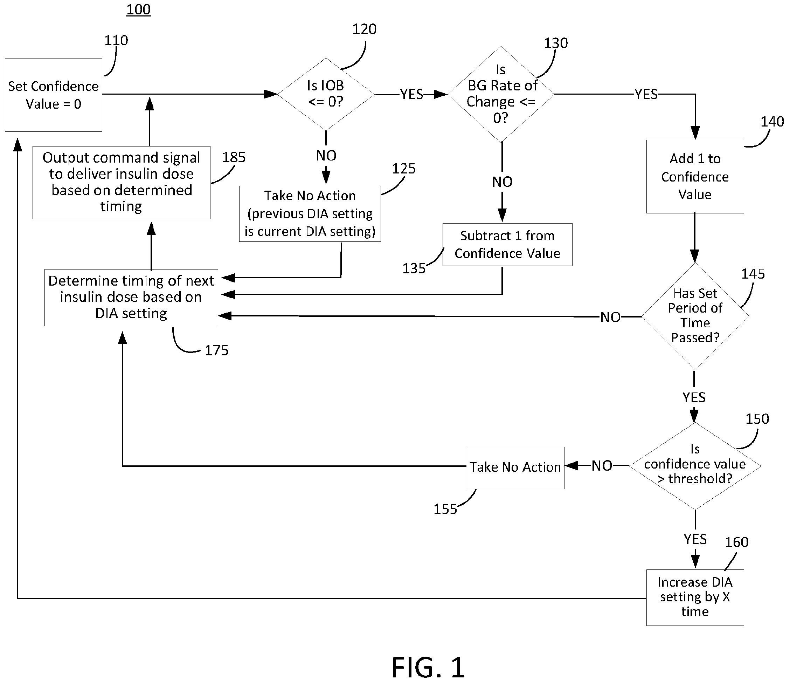

An example provides a process that may be used with any additional algorithms or computer applications, which manage blood glucose levels and insulin therapy. Such algorithms may be referred to as automatic medication delivery algorithms and may include an "artificial pancreas" algorithm-based system, or more generally, an artificial pancreas (AP) application, which provides automatic delivery of an insulin based on a blood glucose sensor input, such as that received from a CGM or the like. The types of medications that may be managed by the automatic medication delivery algorithms may include pain relievers, insulin, glucagon, blood pressure medications, blood thinners, or the like. In a specific example, the artificial pancreas (AP) application when executed by a processor may enable a system to monitor a user's glucose values, determine an appropriate level of insulin for the user based on the monitored glucose values (e.g., blood glucose concentrations or blood glucose measurement values) and other information, such as user-provided information, such as carbohydrate intake, exercise times, meal times or the like, and take actions to maintain a user's blood glucose value within an appropriate range. The appropriate blood glucose value range may be considered a target blood glucose value of the particular user. For example, a target blood glucose value may be acceptable if it falls within the range of 80 mg/dL to 120 mg/dL, which is a range satisfying the clinical standard of care for treatment of diabetes. However, an AP application as described herein may be able to establish a target blood glucose value more precisely and may set the target blood glucose value at, for example, 110 mg/dL, or the like. As described in more detail with reference to the examples of FIGs. 1-4, the AP application may utilize the monitored blood glucose values and other information to generate and send a command to a medical device including, for example, a pump, to control delivery of insulin to the user, change the amount or timing of future doses, as well as to control other functions based on the profile of duration of insulin action.

In another example, the described processes may be used in a non-AP application-like system as well. For example, as long as the system gets frequent blood glucose readings, the described processes may be employed. For example, the described processes may be reduced to a simple question or prompt presented on a diabetes management device or the like that asks the user, for example, approximately 90 minutes after a meal bolus "Did you go hypoglycemic?" (i.e., did the user's measured blood glucose fall below 70 mg/dL) and "Did you go hyperglycemic?" (i.e., did the user's measured blood glucose go above 120 mg/dL). The responses to the prompt may be helpful in the modification of the duration of insulin action. Similarly, for cases in which the insulin onboard is less than zero, an AP application-like system is unnecessary so long as a user's insulin onboard is known, and frequent blood glucose measurement values are available.

An advantageous solution is proposed in which an example system uses two functions, one for insulin delivered in response to a meal and one for insulin delivered during any other time. The disclosed system, processes and techniques are adaptive or custom, which enables a determination of a duration of insulin action that is particular to the patient or user. While the system may operate under the assumption that all patients have a 3 hour duration of insulin action (DIA) for meals and a 4 hour DIA for other insulin. The DIA may be unique to each individual and values between 3-8 hours may be realistic. In a situation where the assumed DIA is 4 hours and the patient's true DIA is 8 hours, there could be an event where a personal diabetes management application, such as an AP application, has determined there is no insulin onboard; however, the patient may still in fact be under the effects of the delivered insulin. This could lead to a non-ideal situation where the system delivers more insulin than a patient needs due to the fact that the system thinks there is no more insulin remaining in the patient's bloodstream. It could also lead to the patient treating themselves with too much insulin during a bolus for the same reasons.

In the examples, the determination of the largest negative rate of change may use curve fitting techniques. For example, the AP application may utilize an insulin effectiveness profile based on data related to insulin action versus a DIA curve.

The DIA may be the user's estimate of how long the effectiveness of insulin takes to decay and assumes that after insulin delivery 100 % of the insulin is available, however, it usually takes some time, such as 60-90 minutes, for insulin to be used in the body.

At times, it may be beneficial to have two different settings for DIA: 1) A first DIA value when a user is given a bolus (any time - meal time or correction bolus), and 2) A second DIA value any time based on the amount of insulin onboard when the AP Application generates an instruction to deliver insulin. The assumption is that an insulin bolus is used faster, such as 3 hours of DIA, while the insulin delivered based on instructions by the AP application, such as basal dosages - small doses of insulin given every 5 minutes - is used more slowly, such as 4 hours of DIA. The following examples may be used in at least three scenarios -Exercise, meals and regular use - to determine an optimal DIA for those at least three scenarios.

It would be advantageous to provide a system, process and techniques that learn a user's DIA from use of the example system as described herein. The described example processes and example system may track blood glucose values measured by a continuous glucose monitor (CGM) and using the measured blood glucose values in the comparison to insulin onboard (IOB) values. The duration of insulin action (DIA) signifies how long insulin acts on a person, but without the present examples a profile of the effectiveness of the insulin is not established overtime. For example, when insulin is delivered, there is a ramp up time of approximately 1 hour until peak effectiveness of the insulin is reached followed by a ramp down time.

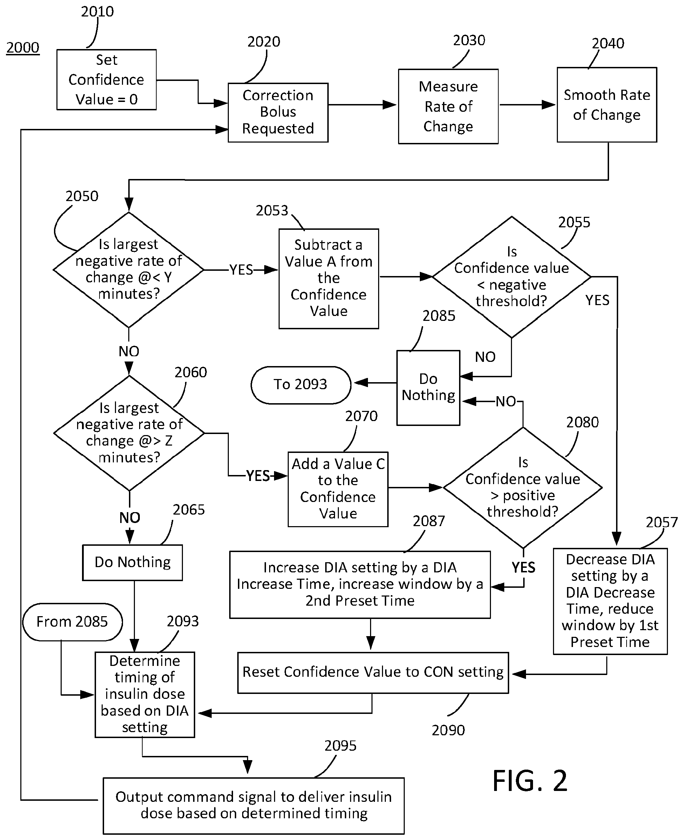

FIGs. 1 and 2 show a flowcharts of process examples for updating a duration of insulin action setting.

In the example of FIG. 1, the process 100 may be implemented by programming code, such as an AP application, which is executed by a processor. The AP application when executed may utilize a user's glucose concentration and insulin delivery values in the determination of insulin action for a respective user. In the example process 100, the AP application may be operable to frequently (e.g., continuously or near-continuously) compare a calculated insulin onboard (IOB) of a user to the rate of change of the user's blood glucose measurement values provided by a continuous glucose monitor (CGM). As a rough approximation, when there is 0 or negative IOB, there should not be a negative BG rate of change. If the rate of change, for example, is consistently negative when there is no IOB, the consistently negative rate of change suggests that the DIA is longer than currently used in the AP application.

In an operational example, at the initiation of the AP application and devices, such as a continuous glucose monitor or sensor, a pump, a personal diabetes management device (each shown in another example), the AP application may set a confidence value equal to zero or some other initiation value (110). This may be a one-time operation that is performed, for example, when a user begins using the AP application, or in a similar situation. The process 100 may proceed to determine at predetermined intervals, whether a user's insulin onboard (IOB) is less than or equal to zero (120). The AP application is operable to allow for a negative IOB. For example, the AP application may enable an indication of -1.88 times basal rate. If no insulin onboard, the blood glucose may either be steady or rising. If the blood glucose rate of change is decreasing, it may mean that insulin is onboard regardless of what is determined at 120.

The time period during which the process 100 determine the amount of IOB is less than or equal to zero may initially be equal to a preset duration of insulin action, such as 3 or 4 hours. In response to a NO determination, the AP application does nothing with regard to adjusting the duration of insulin action (125). For example, the AP application may continue monitoring IOB and other functions. In response to the do nothing at 125, the process 100 may return to 120.

Conversely, in response to a YES determination at 120, the process 100 may proceed to 130 at which the AP application is operable to determine whether the blood glucose (BG) rate of change is less than or equal to zero. In response to a determination that the BG rate of change is not less than or equal to zero (NO), the process 100 proceeds to 135 where the AP application is operable to modify the confidence value by subtracting a value 1 from the confidence value to provide a modified confidence value. The AP application may interpret the increasing BG rate of change as the amount of insulin onboard is diminished which corroborates the determination at 120. The confidence value may be related to a duration of insulin action setting for the user.

In contrast, if the BG rate of change is determined to be decreasing at 130 (YES), the AP application may interpret the decreasing BG rate of change as the amount of insulin onboard is not yet diminished which does not corroborate the determination at 120. As a result of the non-corroboration with 120, the AP application may be operable to proceed to add a value 1 to the confidence value (140) to provide a modified confidence value.

While the change of the confidence value by 1 at 135 or 145, may reflect a value corresponding to the rate of change of the user's blood glucose measurements as received from a continuous blood glucose meter or the like. In an example, the value 1 may be another value that is variable. The value may vary based on the how negative or positive the blood glucose rate of change is. For example, a rate of change of 3.25 may cause a confidence value to be decreased 3.25 (i.e., present confidence value minus 3.25). Similarly, if the rate of change is negative (-) 3.25, the confidence value may be increased by 3.25 (i.e., present confidence value plus 3.25). The closer the confidence value is to zero the more trust there is in the determined DIA, whereas the farther the confidence value is away from zero the less trust there is in the determined DIA. A high confidence value is actually an indication of a lack of confidence in the DIA. The confidence value may be updated every 5 minutes, weekly, monthly or after some other period of time.

After adding to the confidence value, the AP application may be operable to determine, at 145, whether a set period of time has passed. For example, the AP application may have a clock or counter that tracks how long it has been since the DIA was last changed. For example, the set period of time may be 1 week, 10 days, one month, or the like. In an example, the set period of time may correspond to the update of the confidence value particularly when the confidence value is monitored or updated weekly, monthly or after a longer period of time.

At 150, the confidence value may be compared to a confidence threshold value. The confidence threshold value may be a large number such as 288 (i.e., 12 blood glucose measurements per hour by a continuous glucose monitor times 24 hours), 14640 (i.e., 288 times 30 days) or the like. The comparison may be only performed once a week, two weeks, or the like. In an example, the comparison, in addition to being made once a week, may also be made after a meal and when IOB is close to zero, or more rapidly if the DIA was significantly modified in a recent adjustment. The process 100 may be performed after an amount of time has passed since a meal was consumed so digestion does not affect the IOB determination. The comparison at 150 may, for example, be performed one or two times during the day due to the meal timing, while performance at night is more likely because digestion is more likely completed.

In response to a determination the confidence value is less than the confidence threshold value, the AP application may take no action (155) and proceed to 175. At 175, the AP application may determine the timing for delivering a next insulin dose based on the DIA setting. Alternatively, in response to a determination the confidence value is greater than the confidence threshold value, the AP application proceeds to 160, and the confidence value may be reset. At 160, the AP application may modify the DIA setting by increasing the DIA by X time to provide a modified DIA setting. The X time may be 15 minutes, 20 minutes, 30 minutes, 45 minutes, 1 hour, 2 hours or the like. The DIA setting increased by X time may be stored in the memory by the AP application as the current DIA setting for use in calculations by the AP application, such as those performed for step 175. For example, at step 160, the AP application may increase the DIA setting which is stored as a current DIA setting. As a result, in subsequent iterations of the process 100, the AP application would use the increased DIA setting as a current duration of insulin action setting after steps 125, 135, 145, and 155.

After steps 125, 135, 145, 155 and 160, the process 100 may proceed to 175, where the AP application may retrieve a current duration of insulin action setting stored in memory (shown in another example). The duration of insulin action setting may be used in determining a timing of a next insulin dose. Note duration of insulin action setting may be a current duration of insulin action setting after steps 125, 135, 145, and 155.

Using the determined timing of the next insulin dose, the AP application may generate and output a command signal based on the determined timing to a delivery device or pump (shown in another example) (185).

After outputting the command signal, the AP application may return to 120 and the process 100 (other than step 110) may repeat.

In another example, the AP application may compare the rate of change of the measured blood glucose to a time related to when correction boluses are delivered. The AP application may be initialized with an assumption that the peak insulin action happens at around approximatley 1-1.5 hours after a bolus is given, then it can also be assumed that, on average, the largest negative rate of change value may be located approximately 1.5 hours (or approximately 90 minutes) from delivery of the correction bolus. If this period (i.e., approximately 1.5 hours or approximately 90 minutes) is consistently earlier than when the negative rate of change is detected, then the DIA for the user may be decreased. Alternatively, if the period (i.e., approximately 1.5 hours or approximately 90 minutes) is consistently later than when the negative rate of change is detected, then the DIA for the user may be increased.

FIG. 2 shows a flow chart of an example of an alternate process for updating a duration of insulin action setting.

In the process 2000, the AP application may initially set the confidence value at zero (2010). This initialization of the confidence value may be a one-time operation. The AP application may monitor inputs to the personal diabetes management device (shown in another example) for an input requesting delivery of a correction bolus.

A correction bolus may be delivered when a user determines that their blood glucose measurements are too high, and the user wants to bring their blood glucose measurement to a target blood glucose measurement setting.

In response to detecting the request for a correction bolus at, the AP application may receive a number of blood glucose measurement values from a continuous glucose monitor or the like over a period of time. The period of time may be equal to the user's duration of insulin action, such as 3 hours, 4 hours or the like. The AP application may calculate a rate of change of the blood glucose measurement values received from the continuous glucose monitor (2030). The rate of change data over the period of time in the form of a curve may be smoothed using known curve smoothing methods (2040) to reduce the effects of noise and also improve curve fitting for determining a rate of change.

At 2050, the AP application may process the smoothed curve to identify negative rates of change of the blood glucose measurement values obtained from the continuous glucose monitor. A negative rate of change indicates the occurrence of insulin action in reducing blood glucose. The processing performed by the AP application is operable to identify where on the smoothed curve a largest or greatest negative rate of change occurred which may indicate a peak in the insulin action. The processing of the obtained blood glucose measurement values performed by the AP application determines whether the largest negative rate of change occurs prior to a time threshold Y after delivery of the correction bolus. The time threshold Y may be set in minutes or another unit of time and may be a specific time period related to the duration of insulin action. As mentioned, the peak insulin action is estimated to happen approximately 60-90 minutes (or 1-1.5 hours) after a bolus is given. Therefore, the time threshold Y may be approximately 75 minutes, which may be selected to be a time approximately prior to a median time between when peak insulin action may markedly begin and may markedly start to end for a large percentage (e.g., 85%) of diabetics. Alternatively, the time threshold Y may be approximately equal to a time prior to a median of a current DIA period, where a DIA period may, for example, last as long as 3 hours or 4 hours. Alternatively, the time threshold Y may be approximately 50%, approximately 60%, or approximately 80% of estimated peak insulin action time or an approximate range of percentages of an estimated peak insulin action time, such as or 60% - 80%, or the like. It is assumed that insulin values change most rapidly at approximately halfway through the DIA period.

In response to a determination that the largest negative rate of change occurred at less than Y minutes after delivery of the correctio bolus, the AP application may be operable to proceed to 2053 at which the AP application subtracts a value A from the confidence value, where A is a value such as 1. In an example, the value A subtracted from the confidence value may be variable based on how far the minimum rate of change is outside preset boundaries. For example, the preset boundaries may befor example, less than 60 minutes or greater than 150 minutes for a 3 hour DIA, and the minimum rate of change, which is different from the largest negative rate of change, may be, for example, 10 mg/dL/5 minutes for a 3 hour DIA.

After subtracting the value A from the confidence value at 2053, the AP application may evaluate, at 2055, whether the confidence value is less than a negative threshold. In one example, this value A may be - 6, where there may be a consistent pattern of DIA modification found in all boluses for an average day. In other examples, the A value may be -18, or a consistent pattern to be found over approximately 3 days (e.g., -6 times 3), or the like. If the result of the evaluation returns a YES, the confidence value is less than a negative threshold, the AP application proceeds to 2057 at which the AP application may decrease the current DIA setting by a DIA decrease time variable. The DIA decrease time variable may be a preset amount of time, such as 30 minutes, 40 minutes, 1 hour or the like. Alternatively, the DIA decrease time variable may be a time setting that varies based on different parameters, such as an insulin onboard (IOB) calculation, or the like. In another example, in addition to decreasing the DIA by the DIA decrease time variable, the AP application may be operable to reset the Y time threshold prior to which the largest negative rate of change is to occur. For example, The Y time thesholds may be reset by the AP application to time thresholds, such as 60 minutes, 70 minutes or the like.

Upon decreasing the DIA, or alternatively decreasing the DIA and/or reducing the window to measure the rate of change at 2057, the AP application may proceed to step 2090. At 2090, the AP application may reset the confidence value. For example, the confidence value may be reset to zero. In other examples, the confidence value may be set to a non-zero value. For example, the non-zero value may be based on an indication from the AP application or an algorithm of the confidence in the latest DIA change. In a specific example, if the confidence threshold value was reached very quickly, the AP application or algorithm may reset the confidence value to 50% of its current value since AP application indicates that a 30 min change (increase or decrease) may not have been enough of a change in a time for DIA. After resetting the confidence value at 2090, the process 2000 may proceed to step 2093. Upon determining the timing of a next insulin dose based on the decreased DIA setting at 2093, the AP application may generate and output a command signal to deliver the next insulin dose based on the determined timing (2095). After outputting the command signal, the AP application may be operable to return to 2020 to monitor for a request for a correction bolus.

Returning to 2050 of the process 2000, in response to the largest negative rate of change not occurring at less than Y minutes (i.e., the determination at 2050 is NO, the largest negative rate of change did not occur at less than Y minutes), the AP application may proceed to 2060. At 2060, the AP application evaluates the rate of change to determine whether the largest rate of change occurred at a time after Z minutes of delivery of the correction bolus. In an example, Z minutes may be a time period such as approximately 105 minutes. The 105 minutes may an approximate time near the median time between when peak insulin action may substantially begin and may substantially end for a large percentage (e.g., 85%) of diabetics. Alternatively, Z minutes may be equal to an approximate median of a current DIA period, or may be approximately 90%, approximately 105%, or approximately 120% of an estimated peak insulin action time or an approximate range of percentages of peak insulin action time, such as 110% - 120%, or the like of estimated time of peak insulin action.

In response to an evaluation result in which the AP application determines that the largest negative rate of change did not occur at a time greater than Z minutes (i.e., NO), the AP application may be operable to proceed to step 2065 at which no action is taken, and nothing is done to the DIA setting. After taking no action at 2065, the AP application may proceed to step 2093. At step 2093, the AP application may determine timing of a next insulin dose based on the decreased DIA setting. Upon determining the timing of a next insulin dose based on the decreased DIA setting, the AP application may generate and output a command signal to deliver the next insulin dose based on the determined timing (2095). After outputting the command signal at 2095, the AP application may be operable to return to 2020 to monitor for a request for a correction bolus.

Alternatively, at 2060, in response to an evaluation result in which the AP application determines that YES, the largest negative rate of change did occur at a time greater than Z minutes after the delivery of the bolus, the AP application may proceed 2070. The AP application may, for example, add a value C from the confidence value at 2070. In the example, the value C that is added to the confidence value may be a value that varies based on, for example, how far the minimum rate of change is outside preset boundaries, or based on the DIA, a proportion of the DIA, or the like, where C can be a value such as 1 or the like.

After 2070, the AP application may determine whether the confidence value is greater than a positive threshold (2080). In one example, positive threshold value may be 6, where there may be a consistent pattern of DIA modification found in all boluses for an average day. In other examples, this value may be 18 (e.g., 6 times 3 days), a consistent pattern to be found over approximately 3 days, or the like. If the result of the determination by the AP application is YES, the confidence value is greater than the positive threshold, the process 2000 proceeds to 2087. At 2087, the AP application may increase the DIA by a DIA increase time, such as 30 minutes, 40 minutes, 1 hour or the like. In some examples, in addition to increasing the DIA, the AP application may increase the time threshold Z for determining when the largest negative rate of change occurred after delivery of the correction bolus. The time threshold Z may be set in minutes or another unit of time and may be a specific period of time related to the duration of insulin. As mentioned, the peak insulin action time may be estimated to be approximately 60-90 minutes (or 1-1.5 hours) after a bolus is given. Therefore, the time threshold Z may be approximately 105 minutes, which may be selected to be a time at which peak insulin action is markedly ending for a large percentage (e.g., 85%) of diabetics. Different times may be selected for resetting time threshold Z, such as 110 minutes, 120 minutes, or a range, such as 107 minutes to 113 minutes, or the like. Alternatively, the time threshold Z may be approximately 105%, approximately 110%, or approximately 120% of estimated peak insulin action time or an approximate range of percentages, such as or 90% - 120%, or the like of estimated peak insulin action time.

After increasing the DIA (and the time threshold Z depending upon the example) at 2087, the AP application may proceed to step 2090. At 2090, the AP application may reset the confidence value. For example, the confidence value may be reset to zero. In other examples, the confidence value may be set to a non-zero value. For example, the non-zero value may be based on an indication from the AP application or an algorithm of the confidence in the latest DIA change. In a specific example, if the confidence threshold value was reached very quickly, the AP application or algorithm may reset the confidence value to 50% of its current value since AP application indicates that a 30 min change (increase or decrease) may not have been enough of a change in a time for DIA. After resetting the confidence value at 2090, the process 2000 may proceed to step 2093.

At step 2093, the AP application may determine timing of a next insulin dose based on the decreased DIA setting. Upon determining the timing of a next insulin dose based on the decreased DIA setting, the AP application may generate and output a command signal to deliver the next insulin dose based on the determined timing (2095). After outputting the command signal, the AP application may be operable to return to 2020 to monitor for a request for a correction bolus.

Conversely, if the result of the determination by the AP application, at 2080, is NO, the confidence value is not greater than the positive threshold, the process 2000 proceeds to 2085 where the AP application does nothing with respect to DIA (i.e., does not change DIA or the window to determine the largest negative rate of change), and may proceed to 2093.

At step 2093, the AP application may determine timing of a next insulin dose based on the decreased DIA setting. Upon determining the timing of a next insulin dose based on the decreased DIA setting, the AP application may generate and output a command signal to deliver the next insulin dose based on the determined timing (2095). After outputting the command signal, the AP application may be operable to return to 2020 to monitor for a request for a correction bolus.

As discussed above, curve fitting techniques may be used to fit a blood glucose rate of change into boundaries of most negative rate of change profile. With an expected shape function, the AP application may be operable to retrieve simulated curves of expected rate of change from a memory coupled to the processor executing the AP application. The AP application may be operable to compare an actual rate of change based on a user's blood glucose measurements. The AP application may generate a value, such as an R² (i.e., "R squared") value that is indicative of closely the two curves match. The indication may be a value from 1-20, 1-100 or the like depending upon the granularity of the fit. For example, R² is how well a curve fits the data points. If simulated curves do not fit well, the simulated curve may require correction. In a specific example, a 2 hour DIA curve, a 4 hour DIA curve and a 6 hour DIA curve may be retrieved from memory and used in the comparison to the actual blood glucose measurement values to determine the best fitting curve. The best fitting curve may be stored as the "true" representation of the user's DIA.

Alternatively, the best fitting curve may be given a vote. After a period of time, such as tens to hundreds of iterations when bolus dosages are administered, the curve with the most votes may be used to determine an updated DIA.

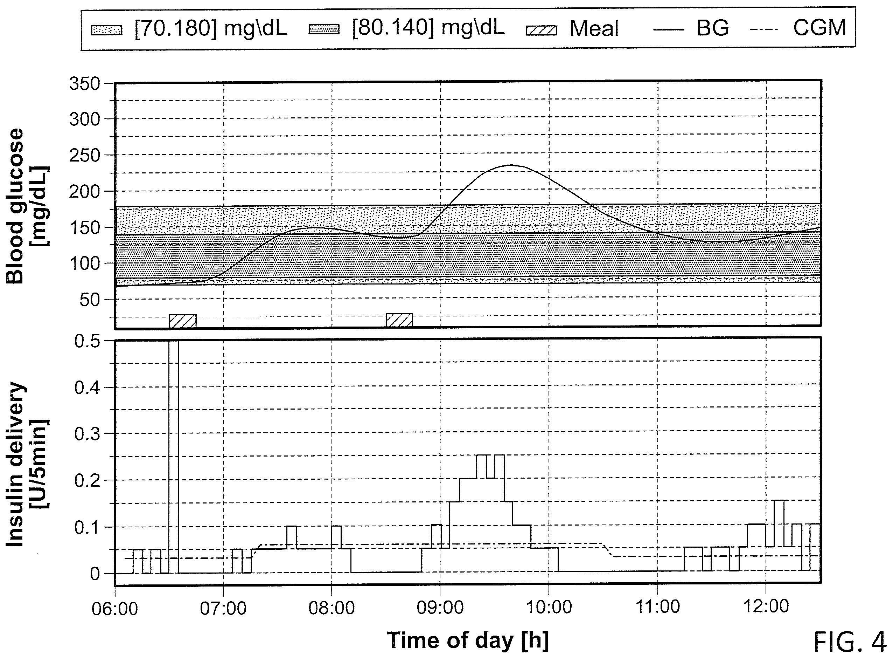

FIG. 4 illustrates an example of a blood glucose measurements (upper chart) and insulin delivery (lower chart). The blood glucose measurement values are shown over a period of twelve hours and the units for the measured blood glucose values are mg/dL. The insulin dosage chart shows the delivered insulin in the vertical axis in units of insulin delivered every 5 minutes, while the horizontal axis is time in hours. The large spike in the insulin delivery chart at approximately 06:30 is a meal bolus.

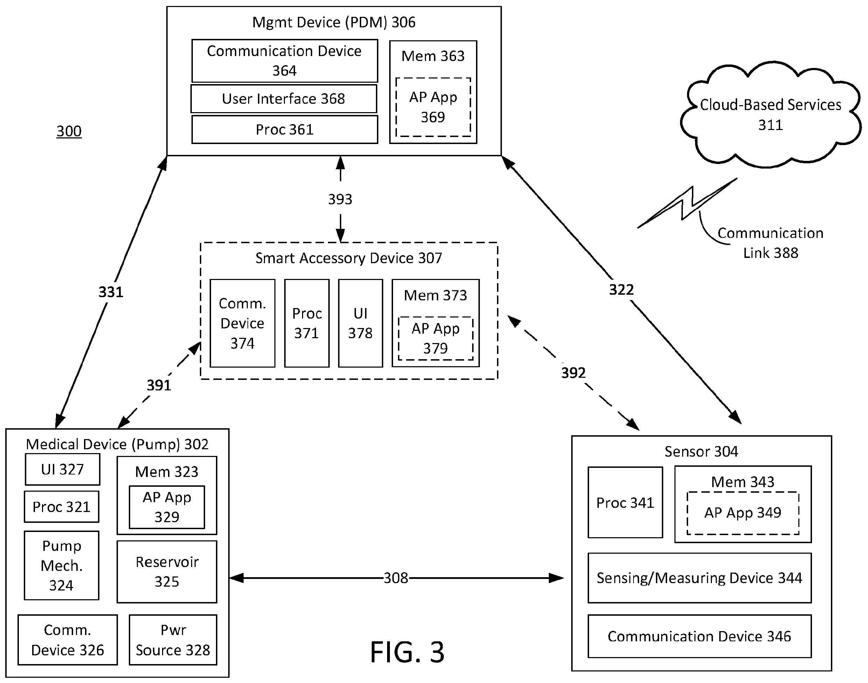

It may be helpful to discuss an example of a drug delivery system that may implement the process example of FIGs. 1 and 2. FIG. 3 illustrates an example of a drug delivery system 300.

The drug delivery system 300 may be operable to implement an AP application that includes functionality to determine a bolus dosage, output an indication of the determined bolus dosage to actuate delivery of the bolus of insulin based on the indication of the determined bolus dosage. The drug delivery system 300 may be an automated drug delivery system that may include a medical device (pump) 302, a sensor 304, and a management device (PDM) 306. The system 300, in an example, may also include a smart accessory device 307, which may communicate with the other components of system 300 either via a wired or wireless communication link.

In an example, the medical device 302 may be attached to the body of a user, such as a patient or diabetic, and may deliver any therapeutic agent, including any drug or medicine, such as insulin or the like, to a user. The medical device 302 may, for example, be a wearable device worn by the user. For example, the medical device 302 may be directly coupled to a user (e.g., directly attached to a body part and/or skin of the user via an adhesive or the like). In an example, a surface of the medical device 302 may include an adhesive to facilitate attachment to a user.

The medical device 302 may include a number of components to facilitate automated delivery of a drug (also referred to as a therapeutic agent) to the user. The medical device 302 may be operable to store the drug and to provide the drug to the user. The medical device 302 is often referred to as a pump, or an insulin pump, in reference to the operation of expelling a drug from the reservoir 325 for delivery to the user. While the examples refer to the reservoir 325 storing insulin, the reservoir 325 may be operable to store other drugs or therapeutic agents, such as morphine or the like, suitable for automated delivery.

In various examples, the medical device 302 may be an automated, wearable insulin delivery device. For example, the medical device 302 may include a reservoir 325 for storing the drug (such as insulin), a needle or cannula (not shown) for delivering the drug into the body of the user (which may be done subcutaneously, intraperitoneally, or intravenously), and a pump mechanism (mech.) 324, or other drive mechanism, for transferring the drug from the reservoir 325, through a needle or cannula (not shown), and into the user. The pump mechanism 324 may be fluidly coupled to reservoir 325, and communicatively coupled to the medical device processor 321. The medical device 302 may also include a power source 328, such as a battery, a piezoelectric device, or the like, for supplying electrical power to the pump mechanism 324 and/or other components (such as the processor 321, memory 323, and the communication device 326) of the medical device 302. Although not shown, an electrical power supply for supplying electrical power may similarly be included in each of the sensor 304, the smart accessory device 307 and the management device (PDM) 306.

The sensor 304 may be operable to detect various analytes, such as lactate, ketones, sodium, potassium, uric acid, alcohol levels, hormones, or the like. In a specific example, the blood glucose sensor 304 may be a device communicatively coupled to the processor 361 or 321 and may be operable to measure a blood glucose value at a predetermined time interval, such as every 5 minutes, or the like. The blood glucose sensor 304 may provide a number of blood glucose measurement values to the AP applications operating on the respective devices.

The medical device 302 may provide insulin the stored in reservoir 325 to the user based on information (e.g., blood glucose measurement values) provided by the sensor 304 and/or the management device (PDM) 306. For example, the medical device 302 may contain analog and/or digital circuitry that may be implemented as a processor 321 (or controller) for controlling the delivery of the drug or therapeutic agent. The circuitry used to implement the processor 321 may include discrete, specialized logic and/or components, an application-specific integrated circuit, a microcontroller or processor that executes software instructions, firmware, programming instructions or programming code (enabling, for example, the artificial pancreas application (AP App) 329 as well as the process examples of FIGs. 1 and 2) stored in memory 323, or any combination thereof. For example, the processor 321 may execute a control algorithm, such as an artificial pancreas application 329, and other programming code that may make the processor 321 operable to cause the pump to deliver doses of the drug or therapeutic agent to a user at predetermined intervals or as needed to bring blood glucose measurement values to a target blood glucose value. In an example, the AP App 329 may include programming code that is operable upon execution by the processor 321 to provide the example processes for adjusting or modifying duration of insulin action settings, confidence values, insulin delivery settings, storing blood glucose measurement values in memory, or the like as described with reference to FIGs. 1 and 2. The size and/or timing of the doses may be programmed, for example, into an artificial pancreas application 329 by the user or by a third party (such as a health care provider, medical device manufacturer, or the like) using a wired or wireless link, such as 331, between the medical device 302 and a management device 306 or other device, such as a computing device at a healthcare provider facility. In an example, the pump or medical device 302 is communicatively coupled to the processor 361 of the management device via the wireless link 331 or via a wireless link, such as 391 from smart accessory device 307 or 308 from the sensor 304. The pump mechanism 324 of the medical device 302 may be operable to receive an actuation signal from the processor 361, and in response to receiving a command signal or actuation signal, expel insulin from the reservoir 325 based on the DIA setting.

In an operational example, the AP application 369 may be executing in the management device 306 and may be operable to control delivery of insulin. For example, the AP application 369 may be operable to determine timing of an insulin dose based on a modified duration of insulin action setting as described with reference to the examples of FIGs. 1 and 2 and may output a command signal to the medical device 302 that actuates the pump mechanism 324 to deliver insulin dose based on the determined timing of the insulin dose.

The other devices in the system 300, such as management device 306, smart accessory device 307 and sensor 304, may also be operable to perform various functions including controlling the medical device 302. For example, the management device 306 may include a communication device 364, a processor 361, and a management device memory 363. The management device memory 363 may store an instance of the AP application 369 that includes programming code, that when executed by the processor 361 provides the process examples described with reference to the examples of FIGs. 1 and 2. The management device memory 363 may also store programming code for providing the process examples described with reference to the examples of FIGs. 1 and 2.

The smart accessory device 307 may be, for example, an Apple Watch^{®}, other wearable smart device, including eyeglasses, provided by other manufacturers, a global positioning system-enabled wearable, a wearable fitness device, smart clothing, or the like. Similar to the management device 306, the smart accessory device 307 may also be operable to perform various functions including controlling the medical device 302. For example, the smart accessory device 307 may include a communication device 374, a processor 371, and a memory 373. The memory 373 may store an instance of the AP application 379 that includes programming code for providing the process examples described with reference to the examples of FIGs. 1 and 2. The memory 373 may also as store programming code and be operable to store data related to the AP application 379. The sensor 304 of system 300 may be a continuous glucose monitor (CGM) as described above, that may include a processor 341, a memory 343, a sensing or measuring device 344, and a communication device 346. The memory 343 may store an instance of an AP application 349 as well as other programming code and be operable to store data related to the AP application 349. The AP application 349 may also include programming code for providing the process examples described with reference to the examples of FIGs. 1 and 2.

Instructions for determining the delivery of the drug or therapeutic agent (e.g., as a bolus dosage) to the user (e.g., the size and/or timing of any doses of the drug or therapeutic agent) may originate locally by the medical device 302 or may originate remotely and be provided to the medical device 302. In an example of a local determination of drug or therapeutic agent delivery, programming instructions, such as an instance of the artificial pancreas application 329, stored in the memory 323 that is coupled to the medical device 302 may be used to make determinations by the medical device 302. In addition, the medical device 302 may be operable to communicate with the cloud-based services 311 via the communication device 326 and the communication link 388.

Alternatively, the remote instructions may be provided to the medical device 302 over a wired or wireless link by the management device (PDM) 306, which has a processor 361 that executes an instance of the artificial pancreas application 369, or the smart accessory device 307, which has a processor 371 that executes an instance of the artificial pancreas application 369 as well as other programming code for controlling various devices, such as the medical device 302, smart accessory device 307 and/or sensor 304. The medical device 302 may execute any received instructions (originating internally or from the management device 306) for the delivery of the drug or therapeutic agent to the user. In this way, the delivery of the drug or therapeutic agent to a user may be automated.

In various examples, the medical device 302 may communicate via a wireless link 331 with the management device 306. The management device 306 may be an electronic device such as, for example, a smart phone, a tablet, a dedicated diabetes therapy management device, or the like. The management device 306 may be a wearable wireless accessory device. The wireless links 308, 331, 322, 391, 392 and 393 may be any type of wireless link provided by any known wireless standard. As an example, the wireless links 308, 331, 322, 391, 392 and 393 may enable communications between the medical device 302, the management device 306 and sensor 304 based on, for example, Bluetooth^{®}, Wi-Fi^{®}, a near-field communication standard, a cellular standard, or any other wireless optical or radio-frequency protocol.

The sensor 304 may be a glucose sensor operable to measure blood glucose and output a blood glucose value or data that is representative of a blood glucose value. For example, the sensor 304 may be a glucose monitor or a continuous glucose monitor (CGM). The sensor 304 may include a processor 341, a memory 343, a sensing/measuring device 344, and communication device 346. The communication device 346 of sensor 304 may include one or more sensing elements, an electronic transmitter, receiver, and/or transceiver for communicating with the management device 306 over a wireless link 322 or with medical device 302 over the link 308. The sensing/measuring device 344 may include one or more sensing elements, such as a glucose measurement, heart rate monitor, or the like. The processor 341 may include discrete, specialized logic and/or components, an application-specific integrated circuit, a microcontroller or processor that executes software instructions, firmware, programming instructions stored in memory (such as memory 343), or any combination thereof. For example, the memory 343 may store an instance of an AP application 349 that is executable by the processor 341.

Although the sensor 304 is depicted as separate from the medical device 302, in various examples, the sensor 304 and medical device 302 may be incorporated into the same unit. That is, in various examples, the sensor 304 may be a part of the medical device 302 and contained within the same housing of the medical device 302 (e.g., the sensor 304 may be positioned within or embedded within the medical device 302). Glucose monitoring data (e.g., measured blood glucose values) determined by the sensor 304 may be provided to the medical device 302, smart accessory device 307 and/or the management device 306 and may be used to determine a bolus dosage of insulin for automated delivery of insulin by the medical device 302.

The sensor 304 may also be coupled to the user by, for example, adhesive or the like and may provide information or data on one or more medical conditions and/or physical attributes of the user. The information or data provided by the sensor 304 may be used to adjust drug delivery operations of the medical device 302.

In an example, the management device 306 may be a computing device operable to manage a personal diabetes treatment plan. The management device 306 may be used to program or adjust operation of the medical device 302 and/or the sensor 304. The management device 306 may be any portable electronic, computing device including, for example, a dedicated controller, such as processor 361, a smartphone, or a tablet. In an example, the management device (PDM) 306 may include a processor 361, a management device management device memory 363, and a communication device 364. The management device 306 may contain analog and/or digital circuitry that may be implemented as a processor 361 (or controller) for executing processes to manage a user's blood glucose levels and for controlling the delivery of the drug or therapeutic agent to the user. The processor 361 may also be operable to execute programming code stored in the management device management device memory 363. For example, the management device management device memory 363 may be operable to store an artificial pancreas application 369 that may be executed by the processor 361. The processor 361 may when executing the artificial pancreas application 369 may be operable to perform various functions, such as those described with respect to the examples in FIGs. 1 and 2. The communication device 364 may be a receiver, a transmitter, or a transceiver that operates according to one or more radio-frequency protocols. For example, the communication device 364 may include a cellular transceiver and a Bluetooth transceiver that enables the management device 306 to communicate with a data network via the cellular transceiver and with the sensor 304 and the medical device 302. The respective transceivers of communication device 364 may be operable to transmit signals containing information useable by or generated by the AP application or the like. The communication devices 326, 346 and 376 of respective medical device 302, sensor 304 and smart accessory device 307 may also be operable to transmit signals containing information useable by or generated by the AP application or the like.

The medical device 302 may communicate with the sensor 304 over a wireless link 308 and may communicate with the management device 306 over a wireless link 331. The sensor 304 and the management device 306 may communicate over a wireless link 322. The smart accessory device 307, when present, may communicate with the medical device 302, the sensor 304 and the management device 306 over wireless links 391, 392 and 393, respectively. The wireless links 308, 331, 322, 391, 392 and 393 may be any type of wireless link operating using known wireless standards or proprietary standards. As an example, the wireless links 308, 331, 322, 391, 392 and 393 may provide communication links based on Bluetooth^{®}, Wi-Fi, a near-field communication standard, a cellular standard, or any other wireless protocol via the respective communication devices 326, 346 and 364. In some examples, the medical device 302 and/or the management device 306 may include a user interface 327, 378 and 368, respectively, such as a keypad, a touchscreen display, levers, buttons, a microphone, a speaker, a display, or the like, that is operable to allow a user to enter information and allow the management device to output information for presentation to the user.

In various examples, the drug delivery system 300 may be an insulin drug delivery system. In various examples, the drug delivery system 300 may implement the artificial pancreas (AP) algorithm (and/or provide AP functionality) to govern or control automated delivery of insulin to a user (e.g., to maintain euglycemia - a normal level of glucose in the blood). The AP application may be implemented by the medical device 302 and/or the sensor 304. The AP application may be used to determine the times and dosages of insulin delivery. In various examples, the AP application may determine the times and dosages for delivery based on information known about the user, such as the user's sex, age, weight, or height, and/or on information gathered about a physical attribute or condition of the user (e.g., from the sensor 304). For example, the AP application may determine an appropriate delivery of insulin based on glucose level monitoring of the user through the sensor 304. The AP application may also allow the user to adjust insulin delivery. For example, the AP application may allow the user to issue (e.g., via an input) commands to the medical device 302, such as a command to deliver an insulin bolus. In some examples, different functions of the AP application may be distributed among two or more of the management device 306, the medical device (pump) 302 or the sensor 304. In other examples, the different functions of the AP application may be performed by one device, such the management device 306, the medical device (pump) 302 or the sensor 304.

As described herein, the drug delivery system 300 or any component thereof, such as the medical device may be considered to provide AP functionality or to implement an AP application. Accordingly, references to the AP application (e.g., functionality, operations, or capabilities thereof) are made for convenience and may refer to and/or include operations and/or functionalities of the drug delivery system 300 or any constituent component thereof (e.g., the medical device 302 and/or the management device 306). The drug delivery system 300 - for example, as an insulin delivery system implementing an AP application - may be considered to be a drug delivery system or an AP application-based delivery system that uses sensor inputs (e.g., data collected by the sensor 304).

In an example, one or more of the devices, 302, 304, 306 or 307 may be operable to communicate via a wireless communication link 388 with cloud-based services 311. The cloud-based services 311 may utilize servers and data storage (not shown). The communication link 388 may be a cellular link, a Wi-Fi link, a Bluetooth link, or a combination thereof, that is established between the respective devices 302, 304, 306 or 307 of system 300. The data storage provided by the cloud-based services 311 may store anonymized data, such as user weight, blood glucose measurements, age, meal carbohydrate information, or the like. In addition, the cloud-based services 311 may process the anonymized data from multiple users to provide generalized information related to the various parameters used by the AP application. For example, an age-based general target blood glucose value may be derived from the anonymized data, which may be helpful when a user first begins using a system such as 300. The cloud-based services 311 may also provide processing services for the system 300, such as performing the process 100 in the example of FIG. 2 or additional processes, such as that described below with reference to FIG. 3.

In an example, the device 302 includes a communication device 364, which as described above may be a receiver, a transmitter, or a transceiver that operates according to one or more radio-frequency protocols, such as Bluetooth, Wi-Fi, a near-field communication standard, a cellular standard, that may enable the respective device to communicate with the cloud-based services 311. For example, outputs from the sensor 304 or the medical device (pump) 302 may be transmitted to the cloud-based services 311 for storage or processing via the transceivers of communication device 364. Similarly, medical device 302, management device 306 and sensor 304 may be operable to communicate with the cloud-based services 311 via the communication link 388.

In an example, the respective receiver or transceiver of each respective device, 302, 306 or 307, may be operable to receive signals containing respective blood glucose measurement values of the number of blood glucose measurement values that may be transmitted by the sensor 304. The respective processor of each respective device 302, 306 or 307 may be operable to store each of the respective blood glucose measurement values in a respective memory, such as 323, 363 or 373. The respective blood glucose measurement values may be stored as data related to the artificial pancreas algorithm or application, such as 329, 349, 369 or 379. In a further example, the AP application, such as 329, 349, 369 or 379, respectively, operating on any of the management device 306, the smart accessory device 307, or sensor 304 may be operable to transmit, via a transceiver implemented by a respective communication device, 364, 374, 346, a control signal for receipt by a medical device. In the example, the control signal may indicate an amount of insulin to be expelled by the medical device 302. While examples may have been described with reference to a particular artificial pancreas algorithm or application, such as 329, 349, 369 or 379, any of the artificial pancreas algorithms or applications 329, 349, 369 and 379, if provided, may be operable to control the delivery of insulin and perform the techniques or examples as described above.

Various operational scenarios and examples of processes performed by the system 300 are described herein. For example, the system 300 may be operable to implement the process examples of FIG. 1A-C. In addition, the system 300 may be operable to implement a process that accounts for periodic advanced TDI adaptivity using past glucose control performance as described with reference to FIG. 3.

The techniques described herein for providing a determination of a new insulin-to-carbohydrate ratio and a new total daily insulin factor for a drug delivery system (e.g., the system 300 or any component thereof) may be implemented in hardware, software, or any combination thereof. For example, the system 300 or any component thereof may be implemented in hardware, software, or any combination thereof. Software related implementations of the techniques described herein may include, but are not limited to, firmware, application specific software, or any other type of computer readable instructions that may be executed by one or more processors. Hardware related implementations of the techniques described herein may include, but are not limited to, integrated circuits (ICs), application specific ICs (ASICs), field programmable arrays (FPGAs), and/or programmable logic devices (PLDs). In some examples, the techniques described herein, and/or any system or constituent component described herein may be implemented with a processor executing computer readable instructions stored on one or more memory components.

Some examples of the disclosed device may be implemented, for example, using a storage medium, a computer-readable medium, or an article of manufacture which may store an instruction or a set of instructions that, if executed by a machine (i.e., processor or microcontroller), may cause the machine to perform a method and/or operation in accordance with examples of the disclosure. Such a machine may include, for example, any suitable processing platform, computing platform, computing device, processing device, computing system, processing system, computer, processor, or the like, and may be implemented using any suitable combination of hardware and/or software. The computer-readable medium or article may include, for example, any suitable type of memory unit, memory, memory article, memory medium, storage device, storage article, storage medium and/or storage unit, for example, memory (including non-transitory memory), removable or non-removable media, erasable or non-erasable media, writeable or re-writeable media, digital or analog media, hard disk, floppy disk, Compact Disk Read Only Memory (CD-ROM), Compact Disk Recordable (CD-R), Compact Disk Rewriteable (CD-RW), optical disk, magnetic media, magnetooptical media, removable memory cards or disks, various types of Digital Versatile Disk (DVD), a tape, a cassette, or the like. The instructions may include any suitable type of code, such as source code, compiled code, interpreted code, executable code, static code, dynamic code, encrypted code, programming code, and the like, implemented using any suitable high-level, low-level, object-oriented, visual, compiled and/or interpreted programming language. The non-transitory computer readable medium embodied programming code may cause a processor when executing the programming code to perform functions, such as those described herein.

Certain examples of the present disclosure were described above. It is, however, expressly noted that the present disclosure is not limited to those examples, but rather the intention is that additions and modifications to what was expressly described herein are also included within the scope of the disclosed examples. Moreover, it is to be understood that the features of the various examples described herein were not mutually exclusive and may exist in various combinations and permutations, even if such combinations or permutations were not made express herein, without departing from the scope of the disclosed examples. In fact, variations, modifications, and other implementations of what was described herein will occur to those of ordinary skill in the art without departing from the scope of the disclosed examples. As such, the disclosed examples are not to be defined only by the preceding illustrative description.

Program aspects of the technology may be thought of as "products" or "articles of manufacture" typically in the form of executable code and/or associated data that is carried on or embodied in a type of machine readable medium. Storage type media include any or all of the tangible memory of the computers, processors or the like, or associated modules thereof, such as various semiconductor memories, tape drives, disk drives and the like, which may provide non-transitory storage at any time for the software programming. It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, inventive subject matter lies in less than all features of a single disclosed example. Thus, the following claims are hereby incorporated into the Detailed Description, with each claim standing on its own as a separate example. In the appended claims, the terms "including" and "in which" are used as the plain-English equivalents of the respective terms "comprising" and "wherein," respectively. Moreover, the terms "first," "second," "third," and so forth, are used merely as labels and are not intended to impose numerical requirements on their objects.

The foregoing description of example examples has been presented for the purposes of illustration and description. It is not intended to be exhaustive or to limit the present disclosure to the precise forms disclosed. Many modifications and variations are possible in light of this disclosure. It is intended that the scope of the present disclosure be limited not by this detailed description, but rather by the claims appended hereto. Future filed applications claiming priority to this application may claim the disclosed subject matter in a different manner and may generally include any set of one or more limitations as variously disclosed or otherwise demonstrated herein.

## Claims

1. A system (300) comprising a management device (306), the device (306) comprising:
a processor (361);
a memory (363) storing programming code and operable to store data, wherein the programming code is executable by the processor;
a user interface (368) and
a transceiver (364) operable to receive and transmit signals containing information usable by or generated by the programming code,
wherein the processor when executing the programming code is operable to control delivery of insulin, and to perform functions to:
- monitor a number of glucose measurements of a user;
- determine (120), at predetermined intervals, a value of a user's insulin onboard;
- in response to a determination that the user's insulin onboard is less than or equal to zero, determine (130) whether a rate of change of the number of glucose measurements is a negative rate of change;
- as a result of a determination that the rate of change of the number of glucose measurements is negative, modify (140) a confidence value by increasing the confidence value, wherein the confidence value is related to a duration of insulin action setting;
- compare (150) the modified confidence value to a confidence value threshold;
- as a result of a determination that the modified confidence value is greater than the confidence value threshold , modify (160) a duration of insulin action setting by increasing the duration of insulin action setting,
- reset (110) the confidence value;
- determine (175) timing of an insulin dose based on the modified duration of insulin action setting; and
- output (185) a command signal to deliver the insulin dose based on the determined timing.

2. The system (300) of claim 1, further comprising a glucose sensor (304) communicatively coupled to the processor, wherein the glucose sensor is operable to:
measure a glucose value at a predetermined time interval; and
provide data indicative of the measured glucose values to the processor.

3. The system (300) of one of the preceding claims, further comprising a pump mechanism (324), wherein the processor is operable to actuate the pump mechanism to deliver the insulin dose based on the determined timing.

4. The system (300) of one of the preceding claims, wherein the processor when executing the programming code is further operable to perform functions to:
- as a result of a determination that the rate of change of the number of glucose measurements is positive, modify (135) the confidence value by reducing the confidence value.

5. A method for operating a management device, comprising:
monitoring a number of glucose measurements of a user;
determining (120), at predetermined intervals, a value of a user's insulin onboard;
in response to a determination that the user's insulin onboard is less than or equal to zero, determining (130) whether a rate of change of the number of glucose measurements is a negative rate of change;
as a result of the determination of the negative rate of change, modifying (140) a confidence value by increasing the confidence value, wherein the confidence value is related to a duration of insulin action setting;
comparing the confidence value to a confidence value threshold;
as a result of a determination that the modified confidence value is greater than the confidence value threshold, modifying (160) a duration of insulin action setting by increasing the duration of insulin action setting;
resetting (110) the confidence value;
determining timing of an insulin dose based on the modified duration of insulin action setting; and
outputting a command signal to deliver an insulin dose based on the determined timing.

6. The method of claim 5, wherein modifying the duration of insulin action setting, comprises:
increasing the duration of insulin action setting by a percentage of an estimated peak time of insulin action.

7. The method of claim 5, further comprising:
as a result of a determination that the rate of change of the number of glucose measurements is positive, modifying (135) the confidence value by reducing the confidence value.

## Patentansprüche

1. System (300), das eine Managementvorrichtung (306) umfasst, wobei die Vorrichtung (306) umfasst:
einen Prozessor (361);
einen Speicher (363), der Programmiercode speichert und betreibbar ist, um Daten zu speichern, wobei der Programmiercode durch den Prozessor ausführbar ist;
eine Benutzeroberfläche (368) und
einen Sendeempfänger (364), der dazu betreibbar ist, Signale zu empfangen und zu senden, die Informationen enthalten, die durch den Programmiercode verwendbar sind oder erzeugt werden,
wobei der Prozessor beim Ausführen des Programmiercodes dazu betreibbar ist, die Insulinabgabe zu steuern und Funktionen auszuführen, zum:
- Überwachen einer Anzahl von Glucosemessungen eines Benutzers;
- Bestimmen (120) eines Wertes von Insulin-on-Board eines Benutzers in vorbestimmten Intervallen;
- in Reaktion auf eine Bestimmung, dass das Insulin-on-Board des Benutzers kleiner oder gleich Null ist, Bestimmen (130), ob eine Änderungsrate der Anzahl von Glucosemessungen eine negative Änderungsrate ist;
- als Ergebnis einer Bestimmung, dass die Änderungsrate der Anzahl von Glucosemessungen negativ ist, Modifizieren (140) eines Konfidenzwerts durch Erhöhen des Konfidenzwerts, wobei der Konfidenzwert mit einer Dauer von Insulinaktivitätseinstellung in Beziehung steht;
- Vergleichen (150) des modifizierten Konfidenzwerts mit einem Konfidenzschwellenwert;
- als Ergebnis einer Bestimmung, dass der modifizierte Konfidenzwert größer als der Konfidenzschwellenwert ist, Modifizieren (160) einer Dauer von Insulinaktivitätseinstellung durch Erhöhen der Dauer der Insulinaktivitätseinstellung;
- Zurücksetzen (110) des Konfidenzwerts;
- Bestimmen (175) des Zeitpunkts einer Insulindosis basierend auf der modifizierten Dauer der Insulinaktivitätseinstellung; und
- Ausgeben (185) eines Befehlssignals zur Abgabe der Insulindosis basierend auf dem bestimmten Zeitpunkt.

2. System (300) nach Anspruch 1, ferner umfassend einen Glucosesensor (304), der kommunikativ mit dem Prozessor gekoppelt ist, wobei der Glucosesensor betreibbar ist zum:
Messen eines Glucosewerts in einem vorbestimmten Zeitintervall; und
Bereitstellen von Daten an den Prozessor, die die gemessenen Glucosewerte angeben.

3. System (300) nach einem der vorhergehenden Ansprüche, ferner umfassend einen Pumpmechanismus (324), wobei der Prozessor betreibbar ist, um den Pumpmechanismus zu betätigen, um die Insulindosis basierend auf dem bestimmten Zeitpunkt abzugeben.

4. System (300) nach einem der vorhergehenden Ansprüche, wobei der Prozessor beim Ausführen des Programmiercodes ferner betreibbar ist zum Durchführen von Funktionen zum:
- Modifizieren (135) des Konfidenzwerts durch Reduzieren des Konfidenzwerts als Ergebnis einer Bestimmung, dass die Änderungsrate der Anzahl der Glucosemessungen positiv ist.

5. Verfahren zum Betreiben einer Managementvorrichtung, das Folgendes umfasst:
Überwachen einer Anzahl von Glucosemessungen eines Benutzers;
Bestimmen (120) eines Wertes von Insulin-on-Board eines Benutzers in vorbestimmten Intervallen;
in Reaktion auf eine Bestimmung, dass das Insulin-on-Board des Benutzers kleiner oder gleich Null ist, Bestimmen (130), ob eine Änderungsrate der Anzahl von Glucosemessungen eine negative Änderungsrate ist;
als Ergebnis der Bestimmung der negativen Änderungsrate Modifizieren (140) eines Konfidenzwerts durch Erhöhen des Konfidenzwerts, wobei der Konfidenzwert mit einer Dauer von Insulinaktivitätseinstellung in Beziehung steht;
Vergleichen des Konfidenzwerts mit einem Konfidenzschwellenwert;
als Ergebnis einer Bestimmung, dass der modifizierte Konfidenzwert größer als der Konfidenzschwellenwert ist, Modifizieren (160) einer Dauer von Insulinaktivitätseinstellung durch Erhöhen der Dauer von Insulinaktivitätseinstellung;
Zurücksetzen (110) des Konfidenzwerts;
Bestimmen des Zeitpunkts einer Insulindosis basierend auf der modifizierten Dauer der Insulinaktivitätseinstellung; und
Ausgeben eines Befehlssignals zur Abgabe der Insulindosis basierend auf dem bestimmten Zeitpunkt.

6. Verfahren nach Anspruch 5, wobei das Modifizieren der Dauer von Insulinaktivitätseinstellung umfasst:
Erhöhen der Dauer von Insulinaktivitätseinestellung um einen Prozentsatz einer geschätzten Höchstzeit der Insulinaktion.

7. Verfahren nach Anspruch 5, ferner umfassend:
Modifizieren (135) des Konfidenzwerts durch Reduzieren des Konfidenzwerts als Ergebnis einer Bestimmung, dass die Änderungsrate der Anzahl der Glucosemessungen positiv ist.

## Revendications

1. Système (300) comprenant un dispositif de gestion (306), le dispositif (306) comprenant :
un processeur (361) ;
une mémoire (363) stockant un code de programmation et pouvant fonctionner pour stocker des données, dans laquelle le code de programmation est exécutable par le processeur ;
une interface utilisateur (368) et
un émetteur-récepteur (364) pouvant fonctionner pour recevoir et émettre des signaux contenant des informations utilisables ou générées par le code de programmation,
dans lequel le processeur, lors de l'exécution du code de programmation, est utilisable pour commander l'administration d'insuline, et pour réaliser des fonctions pour :
- surveiller un nombre de mesures du glucose d'un utilisateur ;
- déterminer (120), à intervalles prédéterminés, une valeur d'insuline encore active d'un utilisateur ;
- en réponse à la détermination que l'insuline encore active de l'utilisateur est inférieure ou égale à zéro, déterminer (130) si un taux de variation du nombre de mesures du glucose est un taux de variation négatif ;
- en résultat à la détermination que le taux de variation du nombre de mesures du glucose est négatif, modifier (140) une valeur de confiance en augmentant la valeur de confiance, la valeur de confiance étant liée à une durée de réglage de l'action de l'insuline ;
- comparer (150) la valeur de confiance modifiée à un seuil de valeur de confiance ;
- en résultat à la détermination que la valeur de confiance modifiée est supérieure au seuil de valeur de confiance, modifier (160) une durée de réglage de l'action de l'insuline par augmentation de la durée du réglage de l'action de l'insuline,
- réinitialiser (110) la valeur de confiance ;
- déterminer (175) le moment d'une dose d'insuline en fonction de la durée modifiée du réglage de l'action de l'insuline ; et
- sortir (185) un signal de commande pour administrer la dose d'insuline en fonction du moment déterminé.

2. Système (300) selon la revendication 1, comprenant en outre un capteur de glucose (304) couplé en communication avec le processeur, dans lequel le capteur de glucose peut fonctionner pour :
mesurer une valeur de glucose à un intervalle de temps prédéterminé ; et
fournir au processeur des données indiquant les valeurs de glucose mesurées.

3. Système (300) selon l'une quelconque des revendications précédentes, comprenant en outre un mécanisme de pompe (324), dans lequel le processeur peut fonctionner pour actionner le mécanisme de pompe pour administrer la dose d'insuline en fonction du moment déterminé.

4. Système (300) selon l'une des revendications précédentes, dans lequel le processeur, lors de l'exécution du code de programmation, peut en outre fonctionner pour réaliser des fonctions pour :
- en résultat à la détermination que le taux de variation du nombre de mesures de glucose est positif, modifier (135) la valeur de confiance en réduisant la valeur de confiance.

5. Procédé de fonctionnement d'un dispositif de gestion, comprenant :
la surveillance d'un nombre de mesures du glucose d'un utilisateur ;
la détermination (120), à intervalles prédéterminés, d'une valeur d'insuline encore active d'un utilisateur ;
en réponse à la détermination que l'insuline encore active de l'utilisateur est inférieure ou égale à zéro, la détermination (130) établissant si un taux de variation du nombre de mesures du glucose est un taux de variation négatif ou non ;
en résultat à la détermination du taux de variation comme étant négatif, la modification (140) d'une valeur de confiance en augmentant la valeur de confiance, la valeur de confiance étant liée à une durée de réglage de l'action de l'insuline ;
la comparaison de la valeur de confiance à un seuil de valeur de confiance ;
en résultat à la détermination que la valeur de confiance modifiée est supérieure au seuil de valeur de confiance, la modification (160) d'une durée de réglage de l'action de l'insuline par augmentation de la durée du réglage de l'action de l'insuline,
la réinitialisation (110) de la valeur de confiance ;
la détermination du moment d'une dose d'insuline en fonction de la durée modifiée du réglage de l'action de l'insuline ; et
la sortie d'un signal de commande pour administrer une dose d'insuline en fonction du moment déterminé.

6. Procédé selon la revendication 5, dans lequel la modification de la durée de réglage de l'action de l'insuline comprend :
l'augmentation de la durée du réglage de l'action de l'insuline d'un pourcentage du temps de pic estimé de l'action de l'insuline.

7. Procédé selon la revendication 5, comprenant en outre :
en résultat à la détermination que le taux de variation du nombre de mesures du glucose est positif, la modification (135) de la valeur de confiance en réduisant la valeur de confiance.
